(19) Europäisches Patentamt

European Patent Office

Office européen des brevets

(11) **EP 0 969 802 B1**

(12) **EUROPÄISCHE PATENTSCHRIFT**

(45) Veröffentlichungstag und Bekanntmachung des Hinweises auf die Patenterteilung:
**29.05.2002 Patentblatt 2002/22**

(21) Anmeldenummer: **98912495.3**

(22) Anmeldetag: **21.03.1998**

(51) Int Cl.⁷: $A61K\ 7/42$, $A61K\ 7/00$

(86) Internationale Anmeldenummer:
**PCT/EP98/01662**

(87) Internationale Veröffentlichungsnummer:
**WO 98/42301 (01.10.1998 Gazette 1998/39)**

(54) **EMULGATORFREIE FEINDISPERSE SYSTEME VOM TYP WASSER-IN-ÖL**

EMULSIFIER-FREE FINELY DISPERSED SYSTEMS OF THE WATER-IN-OIL TYPE

SYSTEMES EN DISPERSION FINE SANS EMULSIFIANTS DE TYPE EAU DANS HUILE

(84) Benannte Vertragsstaaten:
**AT BE CH DE DK ES FI FR GB GR IE IT LI LU MC NL PT SE**

(30) Priorität: **25.03.1997 DE 19712483**

(43) Veröffentlichungstag der Anmeldung:
**12.01.2000 Patentblatt 2000/02**

(73) Patentinhaber: **Beiersdorf Aktiengesellschaft 20245 Hamburg (DE)**

(72) Erfinder:
• **GERS-BARLAG, Heinrich D-25495 Kummerfeld (DE)**
• **MÜLLER, Anja D-23843 Rümpel (DE)**

(56) Entgegenhaltungen:
**EP-A- 0 610 926        EP-A- 0 680 746
EP-A- 0 770 379        EP-A- 0 823 249**

**Beschreibung**

Die vorliegende Erfindung betrifft emulgatorfreie feindisperse Systeme vom Typ Wasser-in-Öl, bevorzugt als kosmetische oder dermatologische Zubereitungen.

Unter Emulsionen versteht man im allgemeinen heterogene Systeme, die aus zwei nicht oder nur begrenzt miteinander mischbaren Flüssigkeiten bestehen, die üblicherweise als Phasen bezeichnet werden. In einer Emulsion ist eine der beiden Flüssigkeiten in Form feinster Tröpfchen in der anderen Flüssigkeit dispergiert.

Sind die beiden Flüssigkeiten Wasser und Öl und liegen Öltröpfchen fein verteilt in Wasser vor, so handelt es sich um eine Öl-in-Wasser-Emulsion (O/W-Emulsion. z. B. Milch). Der Grundcharakter einer O/W-Emulsion ist durch das Wasser geprägt. Bei einer Wasser-in-Öl-Emulsion (W/O-Emulsion z. B. Butter) handelt es sich um das umgekehrte Prinzip, wobei der Grundcharakter hier durch das Öl bestimmt wird.

Um die dauerhafte Dispergierung einer Flüssigkeit in einer anderen zu erreichen, ist bei Emulsionen im herkömmlichen Sinn der Zusatz einer grenzflächenaktiven Substanz (Emulgator) notwendig. Emulgatoren weisen einen amphiphilen Molekülaufbau auf, bestehend aus einem polaren (hydrophilen) und einem unpolaren (lipophilen) Molekülteil, die räumlich voneinander getrennt sind. In einfachen Emulsionen liegen in der einen Phase feindisperse, von einer Emulgatorhülle umschlossene Tröpfchen der zweiten Phase (Wassertröpfchen in W/O- oder Lipidvesikel in O/W-Emulsionen) vor. Emulgatoren setzen die Grenzflächenspannung zwischen den Phasen herab, indem sie an der Grenzfläche zwischen beiden Flüssigkeiten angeordnet sind. Sie bilden an der Phasengrenze Öl/Wasser Grenzflächenfilme aus, wodurch dem irreversiblen Zusammenfließen der Tröpfchen entgegengewirkt wird. Zur Stabilisierung von Emulsionen werden häufig Emulgatorgemische verwendet.

Herkömmliche Emulgatoren können entsprechend ihrem hydrophilen Molekülteil in ionische (anionische, kationische und amphotere) und nichtionische untergliedert werden:

Das wohl bekannteste Beispiel eines anionischen Emulgators ist die Seife, als die man gewöhnlich die wasserlöslichen Natrium- oder Kaliumsalze der gesättigten und ungesättigten höheren Fettsäuren bezeichnet.

Wichtige Vertreter der kationischen Emulgatoren sind die quartemären Ammonium-Verbindungen.

Der hydrophile Molekülteil nichtionischer Emulgatoren besteht häufig aus Glycerin, Polyglycerin, Sorbitanen, Kohlenhydraten bzw. Polyoxyethylenglykolen und ist meistens über Ester- und Etherbindungen mit dem lipophilen Molekülteil verknüpft. Dieser besteht üblicherweise aus Fettalkoholen, Fettsäuren oder Isofettsäuren.

Durch Variation der Struktur und der Größe des polaren und des unpolaren Molekülteils lassen sich Lipophilie und Hydrophilie von Emulgatoren in weiten Grenzen verändern.

Entscheidend für die Stabilität einer Emulsion ist die richtige Auswahl der Emulgatoren. Dabei sind die Charakteristiken aller im System enthaltenen Stoffe zu berücksichtigen. Betrachtet man z. B. Hautpflegeemulsionen, so führen polare Ölkomponenten und beispielsweise UV-Filter zu Instabilitäten. Neben den Emulgatoren werden daher noch andere Stabilisatoren verwendet, die beispielsweise die Viskosität der Emulsion erhöhen und/oder als Schutzkolloid wirken.

Emulsionen stellen einen wichtigen Produkttyp im Bereich kosmetischer und/oder dermatologischer Zubereitungen dar.

Kosmetische Zubereitungen werden im wesentlichen zur Hautpflege benutzt. Hautpflege im kosmetischen Sinn ist in erster Linie, daß die natürliche Funktion der Haut als Barriere gegen Umwelteinflüsse (z.B. Schmutz, Chemikalien, Mikroorganismen) und gegen den Verlust von körpereigenen Stoffen (z.B. Wasser, natürliche Fette, Elektrolyte) gestärkt oder wiederhergestellt wird. Wird diese Funktion gestört, kann es zu verstärkter Resorption toxischer oder allergener Stoffe oder zum Befall von Mikroorganismen und als Folge zu toxischen oder allergischen Hautreaktionen kommen.

Ziel der Hautpflege ist es ferner, den durch tägliches Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Kosmetische Zubereitungen werden auch als Desodorantien verwendet. Solche Formulierungen dienen dazu, Körpergeruch zu beseitigen, der entsteht, wenn der an sich geruchlose frische Schweiß durch Mikroorganismen zersetzt wird.

Medizinische topische Zusammensetzungen enthalten in der Regel ein oder mehrere Medikamente in wirksamer Konzentration. Der Einfachheit halber wird zur eindeutigen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

An sich ist die Verwendung der üblichen Emulgatoren in kosmetischen oder dermatologischen Zubereitungen unbedenklich. Dennoch können Emulgatoren, wie letztlich jede chemische Substanz, im Einzelfalle allergische oder auf Überempfindlichkeit des Anwenders beruhende Reaktionen hervorrufen.

So ist beispielsweise bekannt, daß bestimmte Lichtdermatosen durch gewisse Emulgatoren, aber auch durch verschiedene Fette und gleichzeitige Exposition von Sonnenlicht ausgelöst werden. Solche Lichtdermatosen werden auch "Mallorca-Akne" genannt. Es hat daher nicht an Versuchen gefehlt, die Menge an üblichen Emulgatoren auf ein Minimum, im Idealfall sogar vollständig zu reduzieren.

Eine Reduktion der benötigten Emulgatormenge kann z.B. erreicht werden, wenn ausgenutzt wird, daß feinstverteilte Feststoffteilchen eine zusätzlich stabilisierende Wirkung haben. Dabei kommt es zu einer Anreicherung des festen Stoffes an der Phasengrenze Öl/Wasser in Form einer Schicht, wodurch Zusammenfließen der dispersen Phasen verhindert wird. Von wesentlicher Bedeutung sind dabei nicht die chemischen, sondern die Oberflächeneigenschaften der Feststoffpartikel.

Pickering stellte um 1910 Paraffin-Wasser-Emulsionen her, die nur durch den Zusatz verschiedener Feststoffe wie basisches Kupfersulfat, basisches Eisensulfat oder andere Metallsulfate stabilisiert wurden. Diese Art von Emulsionen wird daher auch als Pickering-Emulsion bezeichnet. Für diese Form von Emulsionen postulierte Pickering die folgenden Bedingungen:

(1) Die Feststoffpartikel sind nur dann zur Stabilisierung geeignet, wenn sie deutlich kleiner sind als die Tröpfchen der inneren Phase und nicht zur Agglomeratbildung neigen.
(2) Wichtige Eigenschaft eines emulsionsstabilisierenden Feststoffs ist außerdem seine Benetzbarkeit. D.h. zur Stabilisierung einer O/W-Emulsion muß der Feststoff beispielsweise leichter von Wasser als von Öl benetzbar sein.

Sozusagen die Urformen von Pickering-Emulsionen tauchten zunächst als unerwünschte Nebeneffekte bei unterschiedlichen technischen Prozessen auf, wie z.B. bei der sekundären Erdölförderung, der Extraktion von Bitumen aus Teersand und anderen Trennungsverfahren, an denen zwei nicht miteinander mischbare Flüssigkeiten und feine, dispergierte Feststoffpartikel beteiligt sind. Im allgemeinen handelt es sich hierbei um W/O-Emulsionen, die durch mineralische Feststoffe stabilisiert werden. Dementsprechend stand die Untersuchung entsprechender Systeme, wie z. B. der Systeme Öl-Wasser-Ruß oder Öl-Wasser-Schieferstaub zunächst im Mittelpunkt der Forschungsaktivitäten.

Grundlegende Untersuchungen haben dabei gezeigt, daß ein Charakteristikum für eine Pickering-Emulsion ist, daß die Feststoffpartikel an der Grenzfläche zwischen den beiden flüssigen Phasen angeordnet sind und dort sozusagen eine mechanische Barriere gegen die Vereinigung der Flüssigkeitströpfchen bilden.

Eine relativ neue technische Entwicklung ist es, Pickering-Emulsionen als Grundlage für kosmetische oder dermatologische Zubereitungen zu verwenden.

Eine Möglichkeit, eine Feststoffstabilisierung in einer kosmetischen oder dermatologischen Zubereitung vorzunehmen, ist nach May-Alert (*Pharmazie in unserer Zeit, 15. Jahrg. 1986, Nr. 1, 1-7*) beispielsweise, Emulgatorgemische zu verwenden, die sowohl anionische als auch kationische Tenside enthalten. Da beim Zusammengeben von Anion- und Kationtensiden unlösliche, elektroneutrale Verbindungen ausfallen, läßt sich durch gezieltes Ausfällen dieser neutralen Tenside in der Grenzfläche Öl/Wasser eine zusätzliche Feststoffstabilisierung im Sinne einer Pickering-Emulsion erreichen.

Die Europäische Offenlegungsschrift 0 686 391 beschreibt Emulsionen vom Typ Wasser-in-Öl, die frei von oberflächenaktiven Substanzen sind und nur durch Feststoffe stabilisiert werden. Zur Stabilisierung werden hier sphärische Polyalkylsilsesquioxan-Partikel eingesetzt, die einen Durchmesser von 100 nm bis zu 20 µm haben. Diese Emulsionen können nach dem oben gesagten als Pickering-Emulsionen bezeichnet werden.

Der Stand der Technik kennt neben den beschriebenen Pickering-Emulsionen weitere emulgatorfreie, feindisperse kosmetische oder dermatologische Zubereitungen, die im allgemeinen als Hydrodispersionen bezeichnet werden. Hydrodispersionen stellen Dispersionen einer flüssigen, halbfesten oder festen inneren (diskontinuierlichen) Lipidphase in einer äußeren wäßrigen (kontinuierlichen) Phase dar. Hydrodispersionen sind, wie im übrigen auch Emulsionen, metastabile Systeme und daher geneigt, in einen Zustand zweier in sich zusammenhängender diskreter Phasen überzugehen.

Bei Hydrodispersionen einer flüssigen Lipidphase in einer äußeren wäßrigen Phase kann die Stabilität beispielsweise dadurch gewährleistet werden, daß in der wäßrigen Phase ein Gelgerüst aufgebaut wird, in welchem die Lipidtröpfchen stabil suspendiert sind. Die Deutsche Offenlegungsschrift 44 25 268 beschreibt stabile feindisperse, emulgatorfreie kosmetische oder dermatologische Zubereitungen vom Typ Öl-in-Wasser, die neben einer Öl- und einer Wasserphase einen oder mehrere Verdicker aus der Gruppe der Acrylsäurepolymere, Polysaccharide und deren Alkylether enthalten, wobei für diese Verdicker eine Grenzflächenspannungsemiedrigung nicht meßbar sein darf.

Basierend auf Hydrodispersionen werden in der Deutschen Offenlegungsschrift 43 03 983 kosmetische oder dermatologische Lichtschutzformulierungen offenbart, welche anorganische Mikropigmente als UV-Filtersubstanzen enthalten. Die Formulierungen bestehen aus einer inneren Lipidphase und einer äußeren wäßrigen Phase und sind im wesentlichen frei von Emulgatoren. Die anorganische Mikropigmente sind in die Lipidphase dieser Hydrodispersion eingearbeitet.

EP-A-610 926 offenbart eine Emulgator -freie, anorganische Mikropigmente enthaltende topische Zubereitung, die jedoch als Hydrodispersion ausgebildet ist.

Aufgabe der vorliegenden Erfindung war, den Stand der Technik um kosmetische oder dermatologische Zubereitungen vom Typ Wasser-in-öl zu bereichern, in welchen auf jeglichen Einsatz von Emulgatoren herkömmlicher Art verzichtet werden kann.

Erstaunlicherweise wird diese Aufgabe gelöst durch

emulgatorfreie kosmetische oder dermatologische Zubereitungen, die feindisperse Systeme vom Typ Wasser-in-Öl darstellen, enthaltend

1. eine Ölphase,
2. eine Wasserphase und
3. mindestens einen Typ mikronisierter, anorganischer Pigmente, die

    a) eine mittlere Partikelgröße von weniger als 200 nm haben und deren Partikel

    b) sowohl hydrophile als auch lipophile Eigenschaften zeigen, die also amphiphilen Charakter besitzen und sich daher an der Grenzfläche Wasser/Öl anordnen und die

    c) gewählt werden aus der Gruppe der Metalloxide, die

    d) gegebenenfalls oberflächlich beschichtet sind,

sowie

gegebenenfalls weitere kosmetische oder pharmazeutische Hilfs-, Zusatz- und/oder Wirkstoffe enthaltend.

Der amphiphile Charakter der erfindungsgemäßen anorganischen Pigmente zeigt sich beispielsweise darin, daß diese sowohl in Wasser als auch in Öl dispergierbar sind.

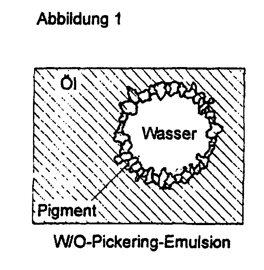

Die erfindungsgemäßen Zubereitungen stellen Abmischungen aus Ölen bzw. öllöslichen Substanzen und Wasser bzw. wasserlöslichen Komponenten dar, die durch den Zusatz der mikronisierten Feststoffpartikel stabilisiert werden und keinen Emulgator im herkömmlichen Sinn enthalten. Eine Erklärungsmöglichkeit für die Stabilität dieser Zubereitungen ist, daß sich die Pigmentpartikel - wie in Abbildung 1 schematisch dargestellt - an die Tröpfchen der dispersen Phase anlagern und sozusagen eine mechanische Barriere bilden, die das Koaleszieren, d. h. das Zusammenfließen der Tröpfchen, verhindert.

Die erfindungsgemäßen Zubereitungen sind in jeglicher Hinsicht überaus befriedigende Präparate, die erstaunlicherweise höchst variabel in ihrem Wasser-Fettphasenverhältnis sind.

Vorteilhaft ist es, den mittleren Partikeldurchmesser der verwendeten Pigmente zwischen 1 nm und 200 nm, besonders vorteilhaft zwischen 5 nm und 100 nm zu wählen.

Der Wasserphasenanteil der erfindungsgemäßen Formulierungen wird vorzugsweise aus dem Bereich von 0,5 bis 75 Gew.-% gewählt, bezogen auf das Gesamtgewicht der Formulierungen.

Im wesentlichen unerheblich für die vorliegende Erfindung ist es, in welcher der gegebenenfalls natürlich vorkommenden Modifikationen die Metalloxide vorliegen.

Vorteilhaft im Sinne der vorliegenden Erfindung ist es, zur Stabilisierung der Pickering-Emulsionen unbehandelte, nahezu reine Pigmentpartikel zu verwenden, insbesondere solche, welche auch als Farbstoff in der Lebensmittelindustrie und/oder als Absorber von UV-Strahlung in Sonnenschutzmitteln verwendet werden können. Vorteilhaft sind beispielsweise die bei der Firma Merck erhältlichen Zinkoxid-Pigmente sowie solche, die unter den Handelsbezeichnungen Zinkoxid neutral bei Haarmann & Reimer oder NanoX von der Harcros Chemical Group erhältlich sind.

Pickering-Emulsionen im Sinne der vorliegenden Erfindung werden vorteilhaft durch anorganische Pigmente stabilisiert, die oberflächlich wasserabweisend behandelt ("gecoatet") sind, wobei gleichzeitig der amphiphile Charakter gebildet werden bzw. erhalten bleiben soll. Diese Oberflächenbehandlung kann darin bestehen, daß die Pigmente nach an sich bekannten Verfahren mit einer dünnen hydrophoben Schicht versehen werden.

Ein solches Verfahren, das im folgenden am Beispiel von Titandioxid beschrieben wird, besteht beispielsweise darin, daß die hydrophobe Oberflächenschicht nach einer Reaktion gemäß

$$n\ TiO_2 + m\ (RO)_3Si\text{-}R' \rightarrow n\ TiO_2\ (oberfl.)$$

erzeugt wird. n und m sind dabei nach Belieben einzusetzende stöchiometrische Parameter, R und R' die gewünschten organischen Reste. Besonders vorteilhaft sind $TiO_2$-Pigmente, beispielsweise die mit Aluminiumstearat beschichteten, unter der Handelsbezeichnung MT 100 T bei der Firma TAYCA erhältlichen.

Eine weitere vorteilhafte Beschichtung der anorganische Pigmente besteht aus Dimethylpolysiloxan (auch: Dimethicone), einem Gemisch vollmethylierter, linearer Siloxanpolymere, die endständig mit Trimethylsiloxy-Einheiten blockiert sind. Besonders vorteilhaft im Sinne der vorliegenden Erfindung sind Zinkoxid-Pigmente, die auf diese Weise beschichtet werden.

Vorteilhaft ist ferner eine Beschichtung der anorganischen Pigmente mit einem Gemisch aus Dimethylpolysiloxan, insbesondere Dimethylpolysiloxan mit einer durchschnittlichen Kettenlänge von 200 bis 350 Dimethylsiloxan-Einheiten, und Silicagel, welches auch als Simethicone bezeichnet wird. Es ist insbesondere von Vorteil, wenn die anorganischen Pigmente zusätzlich mit Aluminiumhydroxid bzw. Aluminiumoxidhydrat (auch: Alumina, CAS-Nr.: 1333-84-2) beschichtet sind. Besonders vorteilhaft sind Titandioxide, die mit Simethicone und Alumina beschichtet sind, wobei die Beschichtung auch Wasser enthalten kann. Ein Beispiel hierfür ist das unter dem Handelsnamen Eusolex T2000 bei der Firma Merck erhältliche Titandioxid.

Vorteilhaft im Sinne der vorliegenden Erfindung ist ferner die Verwendung einer Mischung verschiedener Pigmenttypen sowohl innerhalb eines Kristalls, beispielsweise als Eisenmischoxid oder Talkum (Magnesiumsilicat), als auch durch Kombination mehrerer Metalloxidtypen innerhalb einer Zubereitung. Besonders vorteilhaft sind Magnesiumsilicate, beispielsweise die unter der Handelsbezeichnung Talkum Micron bei der Firma Grolmann erhältlichen.

Es ist ferner vorteilhaft, wenngleich nicht zwingend, die erfindungsgemäßen amphiphilen anorganischen Mikropigmente mit weiteren Pigmenten zu kombinieren, beispielsweise mit Titandioxidpigmenten, die mit Octylsilanol beschichtet sind, und/oder mit Siliciumdioxidpartikeln, die oberflächlich wasserabweisend behandelt sind. Zur Kombination geeignete Siliciumdioxidpartikel sind beispielsweise sphärische Polyalkylsilsesquioxan-Partikel wie sie in der Europäischen Offenlegungsschrift 0 686 391 erwähnt werden. Solche Polyalkylsilsesquioxan-Partikel sind beispielsweise unter den Handelsbezeichnungen Aerosil R972 und Aerosil 200V bei der Firma Degussa erhältlich. Geeignete Titandioxidpartikel sind unter der Handelsbezeichnung T805 ebenfalls bei der Firma Degussa erhältlich.

Vorteilhaft in allen vorgenannten Fällen ist es, die Konzentration der erfindungsgemäßen amphiphilen Pigmente größer als 0,1 Gew.-%, besonders vorteilhaft zwischen 0,1 Gew.-% und 30 Gew.-%, bezogen auf das Gesamtgewicht der Zubereitungen, zu wählen.

Die erfindungsgemäßen Pickering-Emulsionen können als Grundlage für kosmetische oder dermatologische Formulierungen dienen. Diese können wie üblich zusammengesetzt sein und beispielsweise zur Behandlung und der Pflege der Haut, als Lippenpflegeprodukt, als Deoprodukt und als Schmink- bzw. Abschminkprodukt in der dekorativen Kosmetik oder als Lichtschutzpräparat dienen. Zur Anwendung werden die erfindungsgemäßen kosmetischen und dermatologischen Zubereitungen in der für Kosmetika üblichen Weise auf die Haut in ausreichender Menge aufgebracht.

Entsprechend können kosmetische oder topische dermatologische Zusammensetzungen im Sinne der vorliegenden Erfindung, je nach ihrem Aufbau, beispielsweise verwendet werden als Hautschutzcrème, Reinigungsmilch, Sonnenschutzlotion, Nährcrème, Tages- oder Nachtcreme usw. Es ist gegebenenfalls möglich und vorteilhaft, die erfindungsgemäßen Zusammensetzungen als Grundlage für pharmazeutische Formulierungen zu verwenden.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, weichmachende, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösemittel oder Silikonderivate.

Erfindungsgemäße Pickering-Emulsionen können auch Verdickungsmittel enthalten, um die taktilen Eigenschaften der Emulsion zu verbessern.

Insbesondere können die erfindungsgemäßen Pickering-Emulsionen auch Antioxidantien enthalten. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D, L-Carnosin, D-Carnosin, L-Carnosin und deren Derivate (z.B. Anserin), Carotinoide, Carotine (z.B. $\alpha$-Carotin, $\beta$-Carotin, Lycopin) und deren Derivate, Chlorogensäure und deren Derivate, Uponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, $\gamma$-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), femer (Metall)-Chelatoren (z.B. $\alpha$-Hydroxyfettsäuren, Palmitinsäure, Phytinsäure, Lactoferrin), $\alpha$-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. $\gamma$-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO$_4$) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den erfindungsgemäßen Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%. bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Günstig sind auch kosmetische und dermatologische Zubereitungen, die in der Form eines Sonnenschutzmittels vorliegen. Vorzugsweise enthalten diese mindestens eine UV-A-Filtersubstanz und/oder mindestens eine UV-B-Filtersubstanz und/oder mindestens ein weiteres anorganisches Pigment aus der Gruppe der Oxide des Eisens, Zirkoniums, Siliciums, Mangans, Aluminiums, Cers und Mischungen davon sowie Abwandlungen, bei denen die Oxide die aktiven Agentien sind.

Es ist aber auch vorteilhaft im Sinne der vorliegenden Erfindungen, solche kosmetischen und dermatologischen Zubereitungen zu erstellen, deren hauptsächlicher Zweck nicht der Schutz vor Sonnenlicht ist, die aber dennoch einen Gehalt an UV-Schutzsubstanzen enthalten. So werden beispielsweise in Tagescrèmes gewöhnlich UV-Abzw. UV-B-Filtersubstanzen eingearbeitet.

Vorteilhaft können erfindungsgemäße Zubereitungen Substanzen enthalten, die UV-Strahlung im UV-B-Bereich absorbieren, wobei die Gesamtmenge der Filtersubstanzen z. B. 0,1 Gew.-% bis 30 Gew.-%, vorzugsweise 0,5 bis 10 Gew.-%, insbesondere 1,0 bis 6,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen.

Enthalten die erfindungsgemäßen Emulsionen UV-B-Filtersubstanzen, können diese öllöslich oder wasserlöslich sein. Erfindungsgemäß vorteilhafte öllösliche UV-B-Filter sind z.B.:

- 3-Benzylidencampher-Derivate, vorzugsweise 3-(4-Methylbenzyliden)campher, 3-Benzylidencampher;
- 4-Aminobenzoësäure-Derivate, vorzugsweise 4-(Dimethylamino)-benzoësäure(2-ethylhexyl)ester, 4-(Dimethylamino)benzoësäureamylester;
- Ester der Zimtsäure, vorzugsweise 4-Methoxyzimtsäure(2-ethylhexyl)ester, 4-Methoxyzimtsäureisopentylester;
- Ester der Salicylsäure, vorzugsweise Salicylsäure(2-ethylhexyl)ester, Salicylsäure(4-isopropylbenzyl)ester, Salicylsäurehomomenthylester,
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester, -2,4,6-Trianilino-(p-carbo-2'-ethyl-1'-hexyloxy)-1,3,5-triazin.

Vorteilhafte wasserlösliche UV-B-Filter sind z.B.:

- Salze der 2-Phenylbenzimidazol-5-sulfonsäure wie ihr Natrium-, Kalium- oder ihr Triethanolammonium-Salz, sowie die Sulfonsäure selbst;
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und ihre Salze.

Die Liste der genannten UV-B-Filter, die in den erfindungsgemäßen Pickering-Emulsionen verwendet werden können, soll selbstverständlich nicht limitierend sein.

Es kann auch von Vorteil sein, in erfindungsgemäßen Pickering-Emulsionen UV-A-Filter zu verwenden, die bisher üblicherweise in kosmetischen Zubereitungen enthalten sind. Bei diesen Substanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion. Auch Zubereitungen, die UV-A-Filter enthalten, sind Gegenstand der Erfindung. Es können die für die UV-B-Kombination verwendeten Mengen eingesetzt werden.

Vorteilhaft können erfindungsgemäße Zubereitungen außerdem als Grundlage für kosmetische Desodorantien bzw. Antitranspirantien eingesetzt werden, so daß die vorliegende Erfindung in einer besonderen Ausführungsform Pickering-Emulsionen als Grundlage für kosmetische Desodorantien betrifft.

Kosmetische Desodorantien dienen dazu, Körpergeruch zu beseitigen, der entsteht, wenn der an sich geruchlose frische Schweiß durch Mikroorganismen zersetzt wird. Den üblichen kosmetischen Desodorantien liegen unterschiedliche Wirkprinzipien zugrunde.

In sogenannten Antitranspirantien kann durch Adstringentien - vorwiegend Aluminiumsalze wie Aluminiumhydroxychlorid (Aluchlorhydrat) - die Bildung des Schweißes reduziert werden.

Durch die Verwendung antimikrobieller Stoffe in kosmetischen Desodorantien kann die Bakterienflora auf der Haut reduziert werden. Dabei sollten im Idealfall nur die Geruch verursachenden Mikroorganismen wirksam reduziert werden. Der Schweißfluß selbst wird dadurch nicht beeinflußt, im Idealfall wird nur die mikrobielle Zersetzung des Schweißes zeitweilig gestoppt.

Auch die Kombination von Adstringentien mit antimikrobiell wirksamen Stoffen in ein und derselben Zusammensetzung ist gebräuchlich. Antimikrobiell wirksame Stoffe sind Elektrolyte bzw. starke Lewis-Säuren, und die Einarbeitung größerer Mengen von Elektrolyten und/oder Lewis-Säuren in kosmetische und/oder dermatologische Zubereitungen ist im allgemeinen nicht unproblematisch. Erstaunlicherweise sind die erfindungsgemäßen Pickering-Emulsionen auch bei der Verwendung höherer Konzentrationen solcher Wirkstoffe stabil.

Erfindungsgemäße W/O-Pickering-Emulsionen sind erhältlich, indem man zunächst die Pigmente in der Fettphase dispergiert und die Fettphase anschließend mit der Wasserphase vereinigt. Werden die Pigmente zuerst in der Wasserphase dispergiert, so erhält man in der Regel O/W-Pickering-Emulsionen.

Gegenstand der Erfindung ist daher auch ein Verfahren zur Herstellung der erfindungsgemäßen Pickering-Emulsionen, das dadurch gekennzeichnet ist, daß man die amphiphilen anorganischen Pigmente in an sich bekannter Weise in der vorzugsweise flüssigen Fettphase, welche gewünschtenfalls kosmetische oder pharmazeutische Hilfs-, Zusatz- und/oder Wirkstoffe enthält, bei gleichmäßigem Rühren und gegebenenfalls unter Erwärmen dispergiert und während des Homogenisiervorgangs die Wasserphase, welche gewünschtenfalls ebenfalls kosmetische oder pharmazeutische Hilfs-, Zusatz- und/oder Wirkstoffe enthält, mit der Fettphase vermischt.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

**BEISPIELE**

|  | Beispiel 1 | Beispiel 2 | Beispiel 3 | Beispiel 4 |
|---|---|---|---|---|
| Caprylic/Capric-Triglycerid | 20 | 20 | - | 20 |
| Mineralöl | - | - | 20 | |
| Octyldodecanol | 20 | 15 | 20 | 20 |
| Synthetisches Bienenwachs | 2 | | | 2 |
| Konservierungsmittel | 0,5 | 0,5 | 0,5 | 0,5 |
| Butylmethoxydibenzoylmethan | - | 2 | - | - |
| Octyltriazon | - | 4 | - | - |
| 3-(4-Methylbenzyliden)campher | - | 4 | - | - |
| TiO$_2$ (Eusolex T2000) | 4 | 4 | 6 | - |
| ZnO (beschichtet) | - | - | - | 10 |
| Talkum | - | - | 2 | - |
| Aerosil R972 | - | 1 | - | - |
| Glycerin | 10 | 3 | 3 | 3 |
| NaCl | 1 | - | - | - |

(fortgesetzt)

| | Beispiel 1 | Beispiel 2 | Beispiel 3 | Beispiel 4 |
|---|---|---|---|---|
| Phenylbenzimidazolsulfonsäure | - | 4 | - | - |
| NaOH | - | 0,75 | - | - |
| Wasser | ad 100 | ad 100 | ad 100 | ad 100 |

**Patentansprüche**

1. Emulgatorfreie kosmetische oder dermatologische Zubereitungen, die feindisperse Systeme vom Typ Wasser-in-Öl darstellen, enthaltend

    1. eine Ölphase,
    2. eine Wasserphase und
    3. einen oder mehrere Typen mikronisierter, anorganischer Pigmente, die

        a) eine mittlere Partikelgröße von weniger als 200 nm haben und deren Partikel
        b) sowohl hydrophile als auch lipophile Eigenschaften zeigen, die also amphiphilen Charakter besitzen und sich daher an der Grenzfläche Wasser/Öl anordnen und die
        c) gewählt werden aus der Gruppe der Metalloxide, die
        d) gegebenenfalls oberflächlich beschichtet sind,

sowie
gegebenenfalls weitere kosmetische oder pharmazeutische Hilfs-, Zusatz- und/oder Wirkstoffe enthaltend.

2. Zubereitungen nach Anspruch 1, **dadurch gekennzeichnet, daß** der Gehalt der verwendeten anorganischen Pigmente zwischen 0,1 Gew.-% und 30 Gew.-% ist, bezogen auf das Gesamtgewicht der Zubereitungen.

3. Zubereitungen nach Anspruch 1, **dadurch gekennzeichnet, daß** der Partikeldurchmesser der verwendeten anorganischen Pigmente zwischen 5 nm und 100 nm liegt.

4. Zubereitungen nach Anspruch 1, **dadurch gekennzeichnet, daß** das oder die verwendeten anorganischen Pigmente Titandioxid und/oder Zinkoxid sind.

5. Zubereitungen nach Anspruch 1, **dadurch gekennzeichnet, daß** eines der verwendeten anorganischen Pigmente Talkum ist.

6. Zubereitungen nach Anspruch 1, **dadurch gekennzeichnet, daß** die verwendeten anorganischen Pigmente oberflächlich wasserabweisend behandelt sind, wobei der amphiphile Charakter der Pigmente gebildet wird bzw. erhalten bleibt.

7. Zubereitungen nach Anspruch 1, **dadurch gekennzeichnet, daß** die verwendeten anorganischen Pigmente mit Dimethylpolysiloxan und/oder Silicagel und/oder Aluminiumhydroxid und/oder Siliziumdioxid beschichtet sind.

8. Zubereitungen nach Anspruch 1, **dadurch gekennzeichnet, daß** die verwendeten anorganischen Pigmente mit Simethicone und Alumina beschichtete Titandioxidpartikel sind.

9. Zubereitungen nach Anspruch 1, **dadurch gekennzeichnet, daß** neben den amphiphilen anorganischen Pigmente weitere Pigmente enthalten sind.

10. Zubereitungen nach Anspruch 1, enthaltend einen oder mehrere Zusatz- oder Wirkstoffe, gewählt aus der Gruppe der Antioxidantien und/oder UV-Schutzmittel.

11. Zubereitungen nach Anspruch 1, enthaltend einen oder mehrere Zusatz- oder Wirkstoffe, gewählt aus der Gruppe der Adstringentien und/oder antimikrobiell wirksamen Stoffe.

**12.** Verfahren zur Herstellung von Pickering-Emulsionen, **dadurch gekennzeichnet, daß** die amphiphilen anorganischen Pigmente in an sich bekannter Weise in der vorzugsweise flüssigen Fettphase, welche gewünschtenfalls kosmetische oder pharmazeutische Hilfs-, Zusatz- und/oder Wirkstoffe enthält, bei gleichmäßigem Rühren und gegebenenfalls unter Erwärmen dispergiert werden und während des Homogenisiervorgangs die Wasserphase, welche gewünschtenfalls ebenfalls kosmetische oder pharmazeutische Hilfs-, Zusatz- und/oder Wirkstoffe enthält, mit der Fettphase vermischt wird.

**Claims**

**1.** Emulsifier-free cosmetic or dermatological preparations, which are finely dispersed systems of the water-in-oil type, comprising

1. an oil phase,
2. an aqueous phase and
3. one or more types of micronized, inorganic pigments which

a) have an average particle size of less than 200 nm, and whose particles
b) have both hydrophilic and lipophilic properties, i.e. have an amphiphilic character, and thus position themselves at the water/oil interface, and which
c) are selected from the group consisting of metal oxides, which
d) are optionally coated on the surface

and also
optionally comprising further cosmetic or pharmaceutical auxiliaries, additives and/or active substances.

**2.** Preparations according to Claim 1, **characterized in that** the content of the inorganic pigments used is between 0.1% by weight and 30% by weight, based on the total weight of the preparations.

**3.** Preparations according to Claim 1, **characterized in that** the particle diameter of the inorganic pigments used is between 5 nm and 100 nm.

**4.** Preparations according to Claim 1, **characterized in that** the inorganic pigment(s) used are titanium dioxide and/or zinc oxide.

**5.** Preparations according to Claim 1, **characterized in that** one of the inorganic pigments used is talc.

**6.** Preparations according to Claim 1, **characterized in that** the inorganic pigments used have been surface-treated to repel water, the amphiphilic character of the pigments being formed or retained.

**7.** Preparations according to Claim 1, **characterized in that** the inorganic pigments used have been coated with dimethylpolysiloxane and/or silica gel and/or aluminium hydroxide and/or silicon dioxide.

**8.** Preparations according to Claim 1, **characterized in that** the inorganic pigments used are titanium dioxide particles coated with simethicone and alumina.

**9.** Preparations according to Claim 1, **characterized in that** other pigments are present in addition to the amphiphilic inorganic pigments.

**10.** Preparations according to Claim 1, comprising one or more additives or active substances selected from the group consisting of antioxidants and/or UV protectants.

**11.** Preparations according to Claim 1, comprising one or more additives or active substances selected from the group consisting of astringents and/or antimicrobial substances.

**12.** Process for the preparation of Pickering emulsions, **characterized in that** the amphiphilic inorganic pigments are dispersed in a manner known per se in the preferably liquid fatty phase, which, if desired, comprises cosmetic or pharmaceutical auxiliaries, additives and/or active ingredients, with uniform stirring and, where necessary, with heating, and during the homogenization process the aqueous phase, which, if desired, likewise comprises cosmetic or pharmaceutical auxiliaries, additives and/or active ingredients, is mixed with the fatty phase.

**Revendications**

1.  Préparations cosmétiques ou dermatologiques exemptes d'émulsifiants, qui représentent des systèmes finement dispersés du type eau-dans-huile, contenant

    1. une phase huileuse,
    2. une phase aqueuse et
    3. un ou plusieurs types de pigments minéraux micronisés qui

       a) ont une taille moyenne de particule de moins de 200 nm et dont les particules
       b) présentent des propriétés aussi bien hydrophiles que lipophiles, et qui ont donc un caractère amphiphile et se disposent par conséquent à l'interface eau/huile et qui
       c) sont choisis dans le groupe des oxydes métalliques qui
       d) sont éventuellement enrobés en surface,

    et contenant éventuellement d'autres adjuvants, additifs et/ou substances actives, cosmétiques ou pharmaceutiques.

2.  Préparations selon la revendication 1, **caractérisées en ce que** la teneur en les pigments minéraux utilisés est comprise entre 0,1% en poids et 30% en poids, par rapport au poids total des préparations.

3.  Préparations selon la revendication 1, **caractérisées en ce que** le diamètre de particule des pigments minéraux utilisés est compris entre 5 nm et 100 nm.

4.  Préparations selon la revendication 1, **caractérisées en ce que** le ou les pigments minéraux utilisés sont le dioxyde de titane et/ou l'oxyde de zinc.

5.  Préparations selon la revendication 1, **caractérisées en ce que** l'un des pigments minéraux utilisés est le talc.

6.  Préparations selon la revendication 1, **caractérisées en ce que** les pigments minéraux utilisés on été soumis à un traitement hydrofuge en surface, le caractère amphiphile des pigments étant acquis ou conservé.

7.  Préparations selon la revendication 1, **caractérisées en ce que** les pigments minéraux utilisés sont enrobés de diméthylpolysiloxane et/ou de gel de silice et/ou d'hydroxyde d'aluminium et/ou de dioxyde de silicium.

8.  Préparations selon la revendication 1, **caractérisées en ce que** les pigments minéraux utilisés sont des particules de dioxyde de titane enrobées de siméthicone et d'alumine.

9.  Préparations selon la revendication 1, **caractérisées en ce que** d'autres pigments sont contenus en plus des pigments minéraux amphiphiles.

10. Préparations selon la revendication 1, contenant un ou plusieurs additifs ou principes actifs, choisis dans le groupe des antioxydants et/ou des agents anti-UV.

11. Préparations selon la revendication 1, contenant un ou plusieurs additifs ou principes actifs, choisis dans le groupe des astringents et/ou des substances à activité antimicrobienne.

12. Procédé pour la préparation d'émulsions de Pickering, **caractérisé en ce qu'**on disperse sous agitation constante et éventuellement en chauffant les pigments minéraux amphiphiles, d'une façon connue en soi, dans la phase lipidique de préférence liquide, qui contient si on le désire des adjuvants, additifs et/ou substances actives cosmétiques ou pharmaceutiques et, pendant le processus d'homogénéisation, on mélange avec la phase lipidique la phase aqueuse qui contient si on le désire également des adjuvants, additifs et/ou substances actives cosmétiques ou pharmaceutiques.

Abbildung 1

Öl

Wasser

Pigment

W/O-Pickering-Emulsion